# EUROPEAN PATENT SPECIFICATION

(11) **EP 3 873 496 B1**
(45) Date of publication and mention of the grant of the patent: **25.03.2026**
(21) Application number: 19878521.4
(22) Date of filing: 04.11.2019
(51) Int. Cl.: A61K 35/28, A61K 35/12, A61K 35/30, A61K 35/33, C12N 5/00, C12N 5/07, C12N 5/071, A61K 35/22, A61P 9/10, A61P 25/00

(54) **TREATMENT OF CEREBRAL HYPOXIA INCLUDING STROKE, CHRONIC TRAUMATIC ENCEPHALOPATHY, AND TRAUMATIC BRAIN INJURY**
BEHANDLUNG VON ZEREBRALER HYPOXIE, EINSCHLIESSLICH SCHLAGANFALL, CHRONISCHER TRAUMATISCHER ENZEPHALOPATHIE UND TRAUMATISCHER HIRNVERLETZUNG
TRAITEMENT DE L'HYPOXIE CÉRÉBRALE COMPRENANT L'ACCIDENT VASCULAIRE CÉRÉBRAL, L'ENCÉPHALOPATHIE TRAUMATIQUE CHRONIQUE ET LA LÉSION CÉRÉBRALE TRAUMATIQUE

(30) Priority: 04.11.2018 US 201862755553 P
(43) Date of publication of application: 08.09.2021
(73) Proprietor: SpinalCyte LLC, Houston, TX 77289 (US)
(72) Inventor: O'HEERON, Pete, Houston, Texas 77059 (US); ICHIM, Thomas, Houston, Texas 77058 (US)
(74) Representative: J A Kemp LLP
(86) International application number: PCT/US2019/059683
(87) International publication number: WO 2020/093051

(56) References cited:
- WO-A1-2012/004566
- WO-A1-2018/013612
- WO-A1-2018/132594
- WO-A1-2020/146874
- WO-A1-2021/050583
- WO-A1-92/06702
- US-A1- 2006 233 766
- US-A1- 2009 202 500
- US-A1- 2016 375 098
- US-A1- 2018 071 342
- THOMAS E. ICHIM ET AL: "Fibroblasts as a practical alternative to mesenchymal stem cells", JOURNAL OF TRANSLATIONAL MEDICINE, vol. 16, no. 1, 27 July 2018 (2018-07-27), pages 1 - 9, XP055620290, DOI: 10.1186/s12967-018-1536-1
- HUANG HSING-I ET AL: "Multilineage Differentiation Potential of Fibroblast-like Stromal Cells Derived from Human Skin", TISSUE ENGINEERING PART A, vol. 16, no. 5, 1 May 2010 (2010-05-01), US, pages 1491 - 1501, XP055921120, ISSN: 1937-3341, DOI: 10.1089/ten.tea.2009.0431
- DENU RYAN A. ET AL: "Fibroblasts and Mesenchymal Stromal/Stem Cells Are Phenotypically Indistinguishable", vol. 136, no. 2, 1 January 2016 (2016-01-01), CH, pages 85 - 97, XP055828076, ISSN: 0001-5792, Retrieved from the Internet <URL:https://www.karger.com/Article/Pdf/445096> DOI: 10.1159/000445096
- CHUN CHOU ET AL: "Intracerebral transplantation of erythropoietin?producing fibroblasts facilitates neurogenesis and functional recovery in an ischemic stroke model", BRAIN AND BEHAVIOR, vol. 9, no. 5, 28 March 2019 (2019-03-28), pages e01274, XP055705295, ISSN: 2162-3279, DOI: 10.1002/brb3.1274
- ANDERSON JOHNATHON D. ET AL: "Mesenchymal stem cell-based therapy for ischemic stroke", vol. 2, no. 1, 1 November 2016 (2016-11-01), XP055964552, Retrieved from the Internet <URL:http://link.springer.com/content/pdf/10.1186/s41016-016-0053-4.pdf> DOI: 10.1186/s41016-016-0053-4
- "The Biology and Therapeutic Application of Mesenchymal cells", part Chapter 30 2017, JOHN WILEY & SONS, INC., article LUIGI BALDUCCI ET AL: "The difference between mesenchymal stromal cells and fibroblasts", pages: 441 - 455
- DEKOSKY, ST ET AL.: "Interleukm-1 receptor antagonist suppresses neurotrophin response in injured rat brain", ANNALS OF NEUROLOGY, vol. 39, no. 1, January 1996 (1996-01-01), pages 123 - 127, XP055705293
- WERNIG, M ET AL.: "Neurons derived from reprogrammed fibroblasts functionally integrate into the fetal brain and improve symptoms of rats with Parkinson's disease", PROCEEDINGS OF THE NATIONAL ACADEMY OF SCIENCE, vol. 105, no. 15, 15 April 2008 (2008-04-15), pages 5856 - 5867, XP008153592, DOI: 10.1073/pnas.0801677105
- CHOU, PC ET AL.: "Intracerebral transplantation of erythropoietin-producing fibroblasts facilitates neurogenesis and functional recovery in an ischemic stroke model", BRAIN AND BEHAVIOR, vol. 9, no. 5, 28 February 2019 (2019-02-28), pages e01274, XP055705295

## Description

This application claims priority to U.S. Provisional Patent Application Serial No. 62/755,553, filed November 4, 2018, which is incorporated by reference herein in its entirety.

### TECHNICAL FIELD

Embodiments of the disclosure concern at least the fields of cell biology, molecular biology, immunology, and medicine.

### BACKGROUND

Stroke, is classically described as a neurological deficit attributed to an acute focal injury of the central nervous system (CNS) by a vascular cause, either hemorrhagic or infarct related. It is known that stroke is a significant cause of is a major cause of disability and death worldwide [1], and the number of stroke-related deaths is increasing [2]. Between 1990 and 2010, the global incidence rate of stroke seemed to be stable, while other parameters such as the incidence of first stroke, prevalence of stroke, disability-adjusted life-years lost due to stroke, and the number of stroke-related deaths increased by 68, 84, 12, and 26%, respectively. Differences between rates and numbers might reflect variations in population structure, increase in life expectancy, and the global improvement of health care services. In 2030 up to 12 million deaths are expected from stroke [1].Moreover, it is the leading cause of long-term disability [3].

Two main types of stroke are recognized: ischemic and hemorrhagic stroke. Ischemic stroke accounts for over 80% of the total number of strokes. Thrombolysis and/or thrombectomy are the only validated therapeutic strategy for ischemic stroke [4, 5]. Unfortunately, less than 20% of patients receive such treatment due to restrictive inclusion criteria. Besides the direct brain injury resulting from ischemia and reperfusion, there is growing evidence that a rapid evolving sterile inflammation worsens infarct size in the first 1-3 days post stroke, and thus worsens clinical outcome [6]. There is a need in the art for new therapies that will improve clinical outcome and be broadly indicated for patients who suffer from a stroke.

Traumatic brain injury (TBI) is a disruption of function in the brain that results from impact of the cranium or penetrating injury. TBI is heterogeneous in its cause and can be seen as a two-step event: 1) a primary injury, which can be focal or diffuse, caused by mechanical impact, that results in primary pathological events such as hemorrhage and ischemia, tearing of tissue and axonal injuries; 2) a secondary injury such as diffuse inflammation, cell death and gliosis, which is a consequence of the primary one. This secondary injury starts immediately after injury and can continue for weeks, and is thought to involve an active inhibition of neural stem cell activity. Collectively, these events lead to neurodegeneration [7].

Head injuries are described as being open or closed. Open head injuries involve penetration of the scalp and skull by bullets, sharp objects, or skull fractures resulting in laceration of brain tissue. Closed injuries occur when rapid brain acceleration or deceleration results from shaking, crash, falls or other sudden insult. This rapid acceleration or deceleration can damage the brain at the point of contact (coup) or opposite that point (countercoup) [8]. The temporal and frontal lobes are most susceptible to damage, which can involve axon and/or blood vessel tearing. Torn blood vessels can leak and lead to hematomas, contusions, or intracerebral and subarachnoid hemorrhages [9].

TBI can be both acute, occurring recently, as well as of the chronic form resulting from the long term consequences of such acute brain injuries including the effects of inflammation and scarring on the brain tissue. Concussion is described as an immediate, but transient, loss of consciousness accompanied by a short period of amnesia. However, the TBI victim may appear to be dazed, disoriented or confused. A concussion may be accompanied by convulsions, hypotension, fainting and facial pallor. These signs and symptoms are usually short-lived in cases of single, uncomplicated concussion.

Football players and boxers appear to suffer a significantly higher frequency of concussion than athletes in other sports. Professional football players in the National Football League (NFL) have recently brought to the public's attention the long-term consequences of multiple concussions incurred during their playing days. Included as frequently reported signs are loss of cognition, decreased communicative skills, compromised emotional stability, poor coordination, memory loss, dementia and post-mortem changes of brain microstructure [10-12]. Interestingly, studies have shown correlations between football player positions and augmented markers of brain injury [13]

An increasingly prevalent subset of TBI is blast-induced or blast TBI (bTBI). With the increasing use of explosives, including improvised explosive devices (IEDs) in the global war on terrorism, bTBI is also increasing. Such injuries are often referred to as the hallmark injury of the wars in Iraq and Afghanistan, and affect both military and civilian workers in battle zones. Blast injuries are the most common cause of TBI in US soldiers in combat and a major cause of disability among service members. Related to but not limited to TBI are hematomas in the brain. Hematomas are broadly defined as collections of blood outside of blood vessels and when they occur in the brain they are associated with the activation and migration of microglial cells to the location of the injury.

In the U.S., about 1.5 million people per year suffer a traumatic brain injury (TBI), reflecting physical damage to the brain that compromises brain function either temporarily or permanently. Of the total number so injured, some 50,000 die while another 80,000 have some degree of disability. The leading causes of TBI are accidents (auto, bicycle, pedestrian), assault, and sport-related injury.

It is known that a significant amount of patients suffering from TBI possess mild injury, and thus may go undiagnosed immediately after injury. Undiagnosed and untreated TBI presents a risk because some signs and symptoms may be delayed for days to months after injury, and may have significant impact on the patient's physical, emotional, behavioral, social, or family status if untreated, and may result in a functional impairment. Because secondary damage from the injury continues after the initial impact, early treatment (and thus rapid diagnosis), particularly point-of-care treatment, is desirable.

Therapies for brain injuries or disease, including at least TBI, that would reduce the injury infarct size as well as limiting the secondary inflammatory responses are needed in the art.

### BRIEF SUMMARY

The present disclosure is directed to systems, methods, and compositions directed to treatment or prevention of brain injury or medical conditions in an individual in need thereof. In particular embodiments, the treatment induces regeneration in the brain of an individual. Particular embodiments utilize the administration of fibroblasts to an individual for treatment or prevention of brain injury or medical conditions. In specific aspects the fibroblasts are regenerative fibroblasts. The regenerative activity may be inherent to the fibroblasts or they may be manipulated to have increased regenerative activity.?

In some embodiments of the present disclosure, the individual has a brain injury or is at risk for having a brain injury. The administration of the fibroblasts may be given after the individual has been diagnosed with the brain injury or may be administered to the individual before being diagnosed with a brain injury, for example when one skilled in the art reasonably expects the individual has or may at some point in the future have a brain injury. In the latter example, the individual may be administered the fibroblasts prophylactically.

In some embodiments, the brain injury may include a traumatic brain injury (TBI), a stroke or ischemic attack, or may include a tauopathy, for example. Examples of traumatic brain injuries include chronic traumatic encephalopathy, one or more concussions, blast-induced traumatic brain injury, coup-contrecoup brain injury, brain contusion, shaken baby syndrome, a penetrating injury, diffuse axonal injury, second impact injury, locked-in syndrome, or a combination thereof. The individual may have received a brain injury from activities related to their vocation (for example, construction, mining, demolition); from exposure to explosives(for example, an individual in a military or in demolition); playing sports for recreation or as a vocation, such as football or boxing for example; or from a traumatic event that produced a mechanical force to the head of the individual (directly or indirectly), such as a vehicle accident for example. The brain injury may have arisen from any event that would cause trauma to the head to induce a TBI, for example. A brain medical condition may or may not be congenital.

In some embodiments, the individual has a brain injury from a medical condition, such as having suffered a stroke or transient ischemic attack. The stroke may be ischemic or hemorrhagic. The individual having suffered from a stroke or transient ischemic attack may have loss of function in certain areas of the brain. The loss of function may be from the primary event and/or may be from inflammation induced by the primary event.

In particular embodiments, the individual has a medical condition such as a tauopathy, which may be either an acute tauopathy or a chronic tauopathy. Examples of acute tauopathies include traumatic brain injuries, stroke, or transient ischemic attacks. Example of chronic tauopathies include Alzheimer's disease, amyotrophic lateral sclerosis/parkinsonism-dementia complex, argyrophilic grain dementia, British type amyloid angiopathy, cerebral amyloid angiopathy, corticobasal degeneration, Creutzfeldt-Jakob disease, dementia pugilistica, diffuse neurofibrillary tangles with calcification, Down's syndrome, frontotemporal dementia (FTD), frontotemporal dementia with parkinsonism linked to chromosome 17, frontotemporal lobar degeneration, Gerstmann-Straussler-Scheinker disease, Hallervorden-Spatz disease, inclusion body myositis, multiple system atrophy, myotonic dystrophy, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, subacute sclerosing panencephalitis, Tangle only dementia, and multi-infarct dementia. Levels of tau may or may not be measured in the individual to assist in the diagnosis of the individual with an acute or chronic tauopathy.

In particular embodiments of the disclosure, the individual is administered an effective amount of fibroblasts, including to induce regeneration in the brain. The fibroblasts may be from any source, including as non-limiting examples, tissues from skin, foreskin, adipose, peripheral blood, cerebral spinal fluid, bone marrow, placenta, cord blood, Wharton's jelly, or a combination thereof. The fibroblasts may have regenerative activities including, but not limited to, an ability to produce of growth factors, an ability to induce neural stem cell proliferation, an ability to induce a healing response, or a combination thereof. The ability to produce growth factors may include an ability to produce the growth factors including VEGF, VDNF, NGF, PDGF-BB, or a combination thereof, for example. The fibroblasts may express one or more specific markers or proteins including at least one of CD105, CD73, CXCR-4, CD90, CD34, NANOG, OCT-4, SSEA-4, stem cell factor receptor, or a combination thereof. In some embodiments, the fibroblasts may lack the CD45 cell marker.

The fibroblasts may be administered to the individual using any suitable method or administration route. The fibroblasts may be administered by injection, such as with a syringe. The administration may be systemic or may be local to a specific site in the brain. The fibroblasts may also be administered intranasally, intrathecally, and/or intravenously. One skilled in the art may be able to determine the optimal administration route and administration dosage, including amount of fibroblasts and number of administrations.

Any individual receiving the therapy or prophylactic of the present disclosure may be of any age, gender, race, and so forth. The individual may be an adult, child, infant, or *in utero.*

Embodiments of the disclosure include methods of inducing regeneration and/or a healing response in the brain of an individual comprising administering a therapeutically effective amount of fibroblasts to the individual. The individual may or may not have a traumatic brain injury, such as one selected from the group consisting of chronic traumatic encephalopathy, one or more concussions, blast-induced traumatic brain injury, coup-contrecoup brain injury, brain contusion, shaken baby syndrome, a penetrating injury, diffuse axonal injury, second impact injury, locked-in syndrome, and a combination thereof. The individual may or may not have suffered from a stroke (ischemic stroke or a hemorrhagic stroke) and/or a transient ischemic attack. The individual may have a chronic or acute tauopathy. In specific embodiments, circulating levels of total tau, cleaved microtubule-associated tau, phosphorylated tau, and/or tau-A are measured in the individual.

The fibroblasts may possess regenerative activity and the administration may be given to the individual prophylactically. In specific embodiments, the regenerative activity comprises the ability to produce a factor selected from the group consisting of VEGF, BDNF, NGF, PDGF-BB, and a combination thereof. The regenerative activity may comprise the ability to stimulate neural stem cell proliferation. In specific embodiments, the fibroblasts are derived from tissues selected from the group consisting of skin, foreskin, adipose, peripheral blood, cerebral spinal fluid, bone marrow, placenta, cord blood, Wharton's jelly, and a combination thereof. The fibroblasts may express CD105, CD73, CXCR-4, CD90, and/or C73. The fibroblasts may express at least one of the markers selected from the group consisting of NANOG, OCT-4, SSEA-4, stem cell factor receptor, and a combination thereof. The fibroblasts may be isolated by a method comprising the steps of isolating a mammalian cell population and enriching for a subpopulation, wherein the subpopulation expresses a CD45⁻ phenotypic profile to thereby isolate fibroblasts with regenerative properties. The fibroblasts may be administered intranasally, intrathecally, and/or intravenously. They may be administered locally to the brain or systemically. The individual may be provided an additional therapy for a brain injury or stroke and/or a transient ischemic attack or chronic or acute tauopathy. The additional therapy may comprise at least one medical procedure, at least one agent, at least one supportive therapy, or a combination thereof. In specific embodiments, the medical procedure comprises a surgery to remove clotted blood, repair skull fractures, reduce bleeding in the brain, relieve pressure in the skull, or a combination thereof. The agent may be at least one agent selected from the group consisting of anti-anxiety agents, anticoagulants, anticonvulsants, antidepressants, diuretics, muscle relaxants, stimulants, agents to medically-induce a coma, and a combination thereof. The agent may be at least one agent selected from the group consisting of a vaccine for Tau pathologies, an antibody against Tau or Tau related proteins, a microtubule stabilizer, a Tau aggregate inhibitor, an agent that inhibits post-translational modifications of Tau, Hsp90 inhibitors, and a combination thereof. In specific embodiments, the supportive therapy comprises at least one physical therapy, occupational therapy, recreational therapy, speech or communication therapy, psychological counseling, vocational counseling, cognitive therapy, brain stimulation therapy, or a combination thereof.

The foregoing has outlined rather broadly the features and technical advantages of the present disclosure in order that the detailed description that follows may be better understood. Additional features and advantages will be described hereinafter which form the subject of the claims herein. It should be appreciated by those skilled in the art that the conception and specific embodiments disclosed may be readily utilized as a basis for modifying or designing other structures for carrying out the same purposes of the present designs. It should also be realized by those skilled in the art that such equivalent constructions do not depart from the spirit and scope as set forth in the appended claims. The novel features which are believed to be characteristic of the designs disclosed herein, both as to the organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present disclosure.

### BRIEF DESCRIPTION OF THE DRAWINGS

For a more complete understanding of the present disclosure, reference is now made to the following descriptions taken in conjunction with the accompanying drawings.

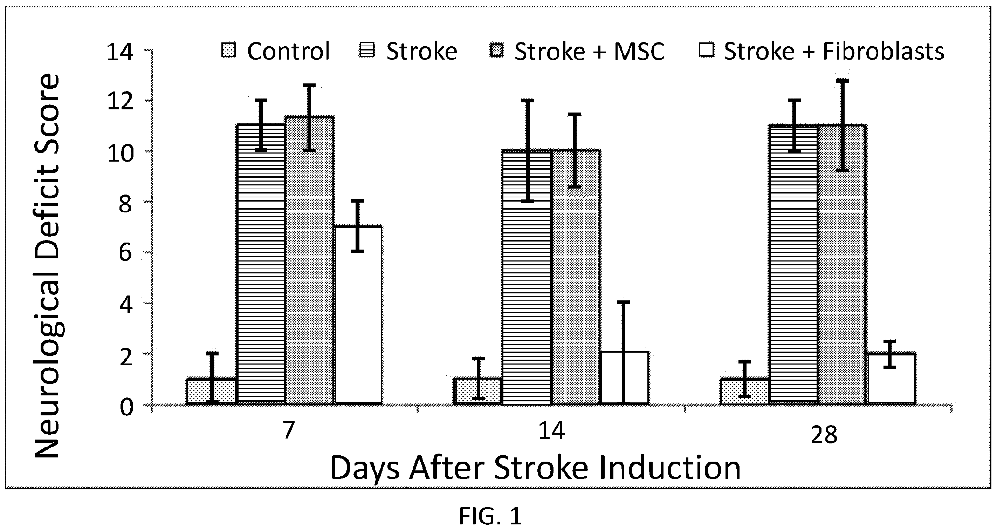

FIG. 1 demonstrates an improvement in neurological deficit score when utilizing fibroblasts instead of mesenchymal stem cells (MSC). In the groupings of four bars, from left to right the bars are as follows: control; stroke; stroke plus MSCs; and stroke plus fibroblasts.

### DETAILED DESCRIPTION

For understanding and describing the practice of the invention, it is to be understood that this invention is not limited to particular embodiments described, as such may, of course, vary. It is also to be understood that the terminology used herein is for the purpose of describing particular embodiments only, and is not intended to be limiting.

### I. Definitions

As used herein the specification. "a" or "an" may mean one or more. As used herein in the claim(s), when used in conjunction with the word "comprising", the words "a" or "an" may mean one or more than one. As used herein "another" may mean at least a second or more. In specific embodiments, aspects of the disclosure may "consist essentially of" or "consist of" one or more elements of the disclosure, for example. Some embodiments of the disclosure may consist of or consist essentially of one or more elements, method steps, and/or methods of the disclosure. It is contemplated that any method or composition described herein can be implemented with respect to any other method or composition described herein. The scope of the present application is not intended to be limited to the particular embodiments of the process, machine, manufacture, composition of matter, means, methods and steps described in the specification. As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z." It is specifically contemplated that x, y, or z may be specifically excluded from an embodiment.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular embodiments, the terms "about" or "approximately" when preceding a numerical value indicates the value plus or minus a range of 15%, 10%, 5%, or 1%. With respect to biological systems or processes, the term can mean within an order of magnitude, preferably within 5-fold, and more preferably within 2-fold, of a value. Unless otherwise stated, the term 'about' means within an acceptable error range for the particular value.

As used herein, the term "administer" or "administration" refers to providing a therapeutically effective amount of a composition to an individual at a site in need. The method of administration may or may not include injection, such as with a syringe, or delivery, such as implantation of the composition at the site in need. Other delivery routes are encompassed in the disclosure and detailed elsewhere herein.

As used herein, "fibroblasts" refers to a population of one or more cells that is (1) adherent to plastic, (2) expresses CD73, and CD105, and (3) possesses an ability to differentiate to at least one of the following three lineages: osteogenic, chondrogenic and adipogenic lineage. Other cells possessing fibroblast-like properties are included within the definition of fibroblasts, with the condition that said cells possess at least one of the following: a) regenerative activity; b) production of growth factors; c) ability to induce a healing response, either directly, or through elicitation of endogenous host repair mechanisms. Fibroblasts can be derived from any tissue including, but not limited to, bone marrow, adipose tissue, amniotic fluid, endometrium, trophoblast-derived tissues, cord blood, Wharton jelly, placenta, amniotic tissue, derived from pluripotent stem cells, and tooth. In some embodiments fibroblasts include cells that are CD34 positive upon initial isolation from tissue but are similar to cells described about phenotypically and functionally. Fibroblasts may include cells that are isolated from tissues using cell surface markers selected from the group consisting of NGF-R, PDGF-R, EGF-R, IGF-R, CD29, CD49a, CD56, CD63, CD73, CD105, CD106, CD140b, CD146, CD271, MSCA-1, SSEA4, STRO-1 and STRO-3 or a combination thereof. The fibroblasts may express the markers either before, during, and/or after expansion.

As used herein, a "healing response" refers to a molecular, cellular, or tissue response in an individual to an administration of a regenerative composition, such as at least one regenerative fibroblast. The healing response may occur at a site of damage resulting from brain injury, including the brain injuries encompassed herein. The response may revert the damaged brain tissue and/or partially or fully restore function in the damaged brain tissue. The response may be driven directly by the regenerative composition and/or the regenerative composition may induce the individual's endogenous repair machinery, such as the immune system, to revert the damage and/or partially or fully restore function in the damaged brain tissue.

The term "pharmaceutically" or "pharmacologically acceptable", as used herein, refer to molecular entities and compositions that do not produce adverse, allergic, or other untoward reactions when administered to an animal or a human.

The term, "pharmaceutically acceptable carrier", as used herein, includes any and all solvents, or a dispersion medium including, but not limited to, water, ethanol, polyol (for example, glycerol, propylene glycol, and liquid polyethylene glycol, and the like), suitable mixtures thereof, and vegetable oils, coatings, isotonic and absorption delaying agents, liposome, commercially available cleansers, and the like. Supplementary bioactive ingredients also can be incorporated into such carriers.

The term "stroke" or "cerebrovascular accident" as used herein refers to neurological symptoms and signs, usually focal and acute, that result from diseases involving blood vessels of the CNS. Generally, strokes are either ischemic strokes, which comprise about 80% of diagnosed stroke patients and result from thrombosis or embolism, or are hemorrhagic strokes, which comprise about 20% of diagnosed stroke patients and result from a vascular rupture in the brain. Strokes involve either the anterior circulation (branches of the internal carotid artery) or the posterior circulation (branches of the vertebral and basilar arteries) of the brain. Infarction occurring in the middle cerebral artery territory causes the most frequently encountered stroke syndrome, with contralateral weakness, sensory loss, and visual field impairment, and depending on the hemisphere involved, either language disturbance or impaired spatial perception. Infarction in the territory of the anterior cerebral artery causes weakness, sensory loss, and urinary incontinence. Damage to the supplementary motor cortex may cause speech disturbance. Bilateral anterior cerebral artery territory infarction, occurring for example when both arteries arise anomalously from a single trunk, may cause severe behavioral disturbance, known as abulia, consisting of profound apathy, motor inertia and muteness. These symptoms are attributable to destruction of inferior frontal lobes of the orbitofrontal cortex. Infarction in the territory of the posterior cerebral artery causes contralateral homonymous hemianopsia by destroying the calcarine cortex. Anterior choroidal artery occlusion can cause contralateral hemiplegia and sensory loss. Infarctions restricted to the structures supplied by the deep penetrating branches of the anterior cerebral artery result in varying combinations of psychomotor slowing, dysarthia, agitation, contralateral neglect, and when left hemispheric, language disturbance. Atherothrombotic vessel occlusion can also occur in the internal carotid artery rather than intracranial vessels. Infarctions may include the territories of both the middle and anterior cerebral arteries with arm and leg weakness and sensory loss. Alternatively, infarctions may be limited to the distal shared territory (border zones of these vessels), producing, by destruction of the motor cortex, weakness limited to the arm or the leg.

As used herein, the term "tauopathy" refers to a number of neurodegenerative diseases that arise from the accumulation of Tau in the brain, including in neurofibrillary or gliofibrillary tangles in the brain. Tauopathies may be formed by the excess accumulation of Tau, misfolding of the Tau protein, the formation of Tau inclusions, post-translational modifications on Tau, or other Tau protein related pathologies. Tauopathies may either be acute tauopathies, which include diseases such as traumatic brain injuries, stroke, or other diseases with an acute accumulation of the Tau protein in the brain. Chronic tauopathies, as used herein, generally refer to conditions associated with a gradual onset of elevated Tau in extracellular fluid of a subject, such as the accumulation of Tau in extracellular fluid, including CSF, ISF, blood, and/or blood fractions (e.g., plasma) over a relatively longer period of time. The relatively long period of time may be on the order of years, or even decades. Chronic tauopathies include, but are not necessarily limited to, Alzheimer's disease, amyotrophic lateral sclerosis/parkinsonism-dementia complex, argyrophilic grain dementia, British type amyloid angiopathy, cerebral amyloid angiopathy, corticobasal degeneration, Creutzfeldt-Jakob disease, dementia pugilistica, diffuse neurofibrillary tangles with calcification, Down's syndrome, frontotemporal dementia (FTD), frontotemporal dementia with parkinsonism linked to chromosome 17, frontotemporal lobar degeneration, Gerstmann-Straussler-Scheinker disease, Hallervorden-Spatz disease, inclusion body myositis, multiple system atrophy, myotonic dystrophy, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, subacute sclerosing panencephalitis, Tangle only dementia, and multi-infarct dementia.

The term "traumatic brain injury" also known as "TBI" is a form of acquired brain injury, which results from trauma-induced damage to the brain. TBI may be described as an injury to the brain caused by an external force. For example, TBI can result when the head suddenly and violently hits an object, such as during a fall, car accident, sporting event, or any number of different ways. A TBI also may result from object piercing the skull and entering brain tissue. Both types of TBI may result in bruised brain tissue, bleeding inside the brain, large or small lacerations in the brain, and/or nerve damage due to shearing forces. The brain can also experience a number of secondary types of damage, such as swelling, fever, seizures, or an imbalance of neurological chemicals. Symptoms of TBI can be mild, moderate, or severe, depending on the extent of the damage to the brain. A person with a mild TBI may remain conscious or may experience a loss of consciousness for a few seconds or minutes. Other symptoms of mild TBI include headache, confusion, lightheadedness, dizziness, blurred vision or tired eyes, ringing in the ears, bad taste in the mouth, fatigue or lethargy, a change in sleep patterns, behavioral or mood changes, and trouble with memory, concentration, attention, or thinking A person with a moderate or severe TBI may show these same symptoms, but may also have a headache that gets worse or does not go away, repeated vomiting or nausea, convulsions or seizures, an inability to awaken from sleep, dilation of one or both pupils of the eyes, slurred speech, weakness or numbness in the extremities, loss of coordination, and increased confusion, restlessness, or agitation. Examples of TBI include, but are not limited to, diffuse axonal injury, concussion, contusion, Coup-Contrecoup injury, Second Impact Syndrome, penetrating injury, Shaken Baby Syndrome, Chronic Traumatic Encephalopathy (CTE), blast-induced traumatic brain injury, and Locked In Syndrome.

The term "transient ischemic attack" (TIA) as used herein refers to a focal brain ischemia that causes sudden neurologic deficits and is not associated with permanent brain infarction (eg, negative results on diffusion-weighted MRI). The diagnosis of TIA may be a clinical diagnosis. Carotid endarterectomy, antiplatelet drugs, and warfarin may decrease a risk of stroke after certain types of TIA. A TIA is similar to an ischemic stroke except that symptoms last <1 h; most TIAs last <5 min. Infarction is very unlikely if deficits resolve within 1 h. Deficits that resolve spontaneously within 1 to 24 h have been shown on diffusion-weighted MRI and other studies to often be accompanied by infarction and are thus no longer considered TIAs. TIAs are most common among the middle-aged and elderly. TIAs markedly increase risk of stroke, beginning in the first 24 h.

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, treatment" can refer to a 10%, 20%, 30%, 40%, 50%, 60%, 70%, 80%, 90%, or 100% reduction in the severity of an established disease or the disease progression, including reduction in the severity of at least one symptom of the disease. For example, a disclosed method for reducing the immunogenicity of cells is considered to be a treatment if there is a detectable reduction in the immunogenicity of cells when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition. In specific embodiments, treatment refers to the lessening in severity or extent of at least one symptom and may alternatively or in addition refer to a delay in the onset of at least one symptom.

### II. Fibroblasts

Embodiments of the present disclosure are directed to systems and methods for the use of fibroblast cells, which may be from autologous, allogeneic, syngeneic, and/or xenogeneic sources. Methods and compositions of the disclosure encompass certain manipulated cells for the treatment of inflammatory degenerative conditions, including those in the brain of an individual. In particular, the cells include at least fibroblasts of any kind. Means of manipulation of fibroblasts are disclosed, as well as fibroblasts of different tissue origins, which actively inhibit degenerative processes in the brain. In one embodiment of the disclosure, fibroblasts are utilized for their ability to inhibit immune responses and also utilized as a cellular therapy for prevention and/or treatment of degenerative brain conditions, including TBI, stroke, transient ischemic attack, and/or acute or chronic tauopathies. In at least one embodiment, fibroblasts are treated with one or more particular agents and/or conditions to be able to directly or indirectly treat and/or prevent brain injuries. In particular embodiments, the agent comprises interferon gamma and/or platelet rich plasma, and in some cases at least interferon gamma and/or platelet rich plasma (and/or platelet rich lysate) can endow the ability of the fibroblasts to directly or indirectly actively suppress immune responses. The route of administration, dosage and frequency is determined as a function of the disease process, as well as stage of the disease, and can be optimized per routine practices in medicine.

In some embodiments of the disclosure, the fibroblasts administered to the individual may be of any origin and be from any tissue type including, for example, skin, foreskin, adipose, peripheral blood, cerebral spinal fluid, bone marrow, placenta, cord blood, Wharton's jelly, or a combination thereof. Methods to isolate fibroblasts are well known in the art, and any such method may be used to obtain fibroblasts. In particular embodiments, the fibroblast expresses at least one marker, which may be, for example, NANOG, OCT-4, SSEA-4, stem cell factor receptor, NGF-R, PDGF-R, EGF-R, IGF-R, CD29, CD49a, CD56, CD63, CD73, CD105, CD106, CD140b, CD146, CD271, MSCA-1, SSEA4, STRO-1, STRO-3, Telomerase, NANOG, Sox2, beta-III-Tubulin, NF-M, MAP2, APP, GLUT, NCAM, NeuroD, Nurr1, GFAP, NG2, Olig1, Alkaline Phosphatase, Vimentin, Osteonectin, Osteoprotegrin, Osterix, Adipsin, Erythropoietin, SM22-alpha, HGF, c-MET, alpha-1-Antriptrypsin, Ceruloplasmin, AFP, PEPCK 1, BDNF, NT-4/5, TrkA, BMP2, BMP4, FGF2. FGF4, PDGF, PGF, TGFalpha, TGFbeta, VEGF or a combination thereof. The fibroblast may be cultured prior to use in the methods of the present disclosure. Any suitable method and culture medium for culturing fibroblasts may be used.

In particular embodiments, allogeneic or autologous fibroblasts are administered to an individual in a non-manipulated manner (for example, without prior exposure to one or more particular agents, such as interferon gamma, and/or conditions) but selected from sources naturally characterized by immune modulatory activity, such as placental fibroblasts or adipose tissue-associated fibroblasts, for example. In other embodiments of the disclosure, any fibroblasts are cultured under conditions capable of inducing retro-differentiation so as to endow an immature phenotype for the fibroblasts, wherein the immature phenotype correlates with enhanced anti-inflammatory and/or immune modulatory potential to induce regeneration and/or a healing response. For example, fibroblasts may be cultured in the presence of one or more histone deacetylase inhibitors, such as valproic acid (Moon *et al.,* 2008; Huang *et al.,* 2011). In addition to HDAC inhibitors, other means of inducing dedifferentiation of the fibroblasts may also be utilized in the context of the current disclosure, such as 8-Br-cAMP (Wang *et al.,* 2011); M-CSF treatment (Li *et al.,* 2016); exposure to reveresine (Li *et al.,* 2016); and/or exposure to stem cell extracts (Xiong *et al.,* 2014). Characterization of fibroblast dedifferentiation can be performed by assessment of extracellular markers, such as CXCR4, VEGFR-2, CD34, and/or CD133, as well as intracellular markers such as SOX-2, NANOG, and/or OCT-4. That is, fibroblasts with regenerative properties have SOX, NANOG OCT4 but have low levels, and upon dedifferentiation they have high levels.

In some embodiments of the disclosure, fibroblast cells that have been dedifferentiated may be utilized for the disclosed methods that express one or more markers selected from the group consisting of NANOG, OCT-4, SSEA-4, stem cell factor receptor, NGF-R, PDGF-R, EGF-R, IGF-R, CD29, CD49a, CD56, CD63, CD73, CD105, CD106, CD140b, CD146, CD271, MSCA-1, SSEA4, STRO-1, STRO-3, Telomerase, NANOG, Sox2, beta-III-Tubulin, NF-M, MAP2, APP, GLUT, NCAM, NeuroD, Nurr1, GFAP, NG2, Olig1, Alkaline Phosphatase, Vimentin, Osteonectin, Osteoprotegrin, Osterix, Adipsin, Erythropoietin, SM22-alpha, HGF, c-MET, alpha-1-Antriptrypsin, Ceruloplasmin, AFP, PEPCK 1, BDNF, NT-4/5, TrkA, BMP2, BMP4, FGF2, FGF4, PDGF, PGF, TGFalpha, TGFbeta, VEGF, and a combination thereof.

In particular embodiments of the disclosure, fibroblasts to be used to induce regeneration and/or healing responses are genetically engineered, for example to express: a) one or more autoantigens; and/or b) one or more immune modulatory proteins. The characteristics of the individual and disease dictate which genes are to be used for engineering of fibroblasts, in at least some cases. In these cases, the autoantigen may be transfected into the fibroblasts in polynucleotide form and the fibroblasts are either cultured to allow for immune modulation or transfected with genes allowing for immune modulation. Genes of particular interest for transfection to induce immune modulation and for inducing regeneration and/or a healing response in the brain include at least the following: Fas ligand, TGF-beta, IL-4, IL-10, HLA-G, indolamine 2,3 deoxygenase, galectin family members, Galectin 3, arginase, and/or IL-20 (de Jesus *et al.,* 2016; Wang *et al.,* 2011; Zhao *et al.,* 2010; Min *et al.,* 2001; Cancedda *et al.,* 2001). Any of the genes described herein or active portions thereof may be cloned into mammalian expression constructs comprising promoter sequences enabling expression in fibroblast cells such as the CMV promoter (Artuc et al., Exp. Dermatol. 1995, 4:317-21). Examples of suitable constructs include, but are not limited to pcDNA3, pcDNA3.1 (+/-), pGL3, PzeoSV2 (+/-), pDisplay, pEF/myc/cyto, pCMV/myc/cyto (each of which is commercially available from vendors such as Invitrogen, for example), or the pSH expression vector that enables a regulated polynucleotide expression in human foreskin cells (Ventura and Villa, 1993, Biochem. Biophys. Commun. 192: 867-9). Examples of retroviral vector and packaging systems are those commercially available from Clontech, San Diego, Calif., USA, including Retro-X vectors pLNCX and pLXSN, which permit cloning into multiple cloning sites and the transgene is transcribed from CMV promoter. Vectors derived from Mo-MuLV are also included such as pBabe, where the transgene will be transcribed from the 5'LTR promoter. After completing plasmid transfection, fibroblasts are harvested by a means allowing for detachment from tissue culture plates, for example, by trypsinization and transferred to a suitable vessel or container for proliferation. Approximately 3 days post-transfection, the cell media is changed to media suitable for proliferation and expansion of modified fibroblasts. One example is Neurobasal A (NBA) proliferation medium comprising Neurobasal-A (Invitrogen), 1% D-glucose (Sigma Aldrich), 1% Penicillin/Streptomycin/Glutamine (Invitrogen), 2% B27 supplement with Retinoic acid (Invitrogen), 0.2% EGF (Peprotech, USA), 0.08% FGF-2 (Peprotech), 0.2% Heparin (Sigma Aldrich, USA) and Valproic acid (Sigma Aldrich) to a concentration of 1 µM. The media is then subsequently changed, such as thrice weekly, and cells are re-plated regularly (for example, 2-8 times up to a maximum of weekly re-plating, becoming more regular as colonies began to develop) to remove non-reprogrammed cells until widespread colony formation is achieved. Various quality control means are known in the art for practitioners of the disclosure to perform clinical administration of the cells. Example criteria for qualification of the cells includes marker identification using means such as flow cytometry, viability, endotoxin content, as well as assessment for microbial and mycoplasma contamination.

In particular embodiments of the disclosure, fibroblasts are cultured *ex vivo* using means known in the art for preserving viability and proliferative ability of fibroblasts. The disclosure provides for the modification of known culture techniques to decrease recognition of fibroblasts by the recipient immune system. In one embodiment fibroblasts are cultured in conditions that lack xenogeneic components, such as fetal calf serum. Xenogeneic components are known to trigger immunological reactions, including elicitation of antibody and T cell reactions (Selvaggi *et al.,* 1997; Mackensen *et al.,* 2000; Kadri *et al.,* 2007; Forni *et al.,* 1976; Lauer *et al.,* 1983). In many individuals, natural antibodies of the IgM isotype exist to fetal calf serum associated components (Irie *et al.,* 1974), causing rejection, inflammation or anaphylaxis subsequent to administration of cells grown in the presence of fetal calf serum (Macy *et al.,* 1989). In specific embodiments, the disclosure encompasses the substitution of fetal calf serum with human platelet rich plasma, platelet lysate, umbilical cord blood serum, autologous serum, and/or defined cytokine mixes as an additional feature to reduce the immunogenicity of fibroblasts. Means of culturing tissues in xenogeneic-free medium are known in the art for other cell types and are incorporated by reference (Riordan *et al.,* 2015).

In one embodiment of the disclosure, fibroblasts are administered to a subject by any suitable route, including by injection (such as intramuscular injection), including in hypoxic areas. Suitable routes include intravenous, subcutaneous, intrathecal, oral, intrarectal, intrathecal, intra-omentral, intraventricular, intrahepatic, and intrarenal.

In certain embodiments, fibroblasts may be derived from tissues comprising skin, heart, blood vessels, bone marrow, skeletal muscle, liver, pancreas, brain, cerebral spinal fluid, adipose tissue, foreskin, placental, Wharton's jelly, and/or umbilical cord. In specific embodiments, the fibroblasts are placental, fetal, neonatal or adult or mixtures thereof.

The number of administrations of cells to an individual will depend upon factors described herein, such as the type and severity of injury, at least in part and may be optimized using routine methods in the art. In specific embodiments, a single administration is required. In other embodiments, a plurality of administration of cells is required. It should be appreciated that the system is subject to variables, such as the particular need of the individual, which may vary with time and circumstances, the rate of loss of the cellular activity as a result of loss of cells or activity of individual cells, and the like. Therefore, it is expected that each individual could be monitored for the proper dosage, and such practices of monitoring an individual are routine in the art.

Embodiments of the disclosure provide methods of reducing immunogenicity of particular types of fibroblasts. Fibroblasts may be derived from various tissues or organs, such as skin, heart, blood vessels, bone marrow, skeletal muscle, liver, pancreas, brain, cerebral fluid, adipose, peripheral blood, placenta, cord blood. Wharton's jelly, and/or foreskin, which can be obtained by biopsy (where appropriate) or upon autopsy. In some aspects, the cells comprise fibroblasts, which can be from a fetal, neonatal, adult origin, or a combination thereof.

In particular embodiments, the isolation procedure utilizes an enzymatic digestion process. Enzymes are used to dissociated tissue to extract cellular populations that are subsequently harvested and grown for isolation of fibroblast cells. Many enzymes are known in the art to be useful for the isolation of individual cells from complex tissue matrices to facilitate growth in culture. A broad range of digestive enzymes for use in cell isolation from tissue is available to the skilled artisan, ranging from weakly digestive (e.g. deoxyribonucleases and the neutral protease, dispase) to strongly digestive (e.g. papain and trypsin), such enzymes are available commercially. A nonexhaustive list of enzymes compatable herewith includes mucolytic enzyme activities, metalloproteases, neutral proteases, serine proteases (such as trypsin, chymotrypsin, or elastase), and deoxyribonucleases. For example, collagenases are known to be useful for isolating various cells from tissues. Deoxyribonucleases can digest single-stranded DNA and can minimize cell-clumping during isolation. Enzymes can be used alone or in combination. Serine protease may be used in a sequence following the use of other enzymes as they may degrade the other enzymes being used. The temperature and time of contact with serine proteases may be monitored. Serine proteases may be inhibited with alpha 2 microglobulin in serum and therefore the medium used for digestion is often serum-free. EDTA and DNase are commonly used and may improve yields or efficiencies. Some methods involve enzymatic treatment with for example collagenase and dispase, or collagenase, dispase, and hyaluronidase, and such methods are provided wherein in certain preferred embodiments, a mixture of collagenase and the neutral protease dispase are used in the dissociating step. Some method employ digestion in the presence of at least one collagenase from Clostridium histolyticum, and either of the protease activities, dispase and thermolysin. Certain method for the present disclosure employ digestion with both collagenase and dispase enzyme activities. Also there are methods which include digestion with a hyaluronidase activity in addition to collagenase and dispase activities. The skilled artisan will appreciate that many such enzyme treatments are known in the art for isolating cells from various tissue sources. For example, the LIBERASE BLENDZYME (Roche) series of enzyme combinations of collagenase and neutral protease are useful and may be used in the instant methods. Other sources of enzymes are known, and the skilled artisan may also obtain such enzymes directly from their natural sources. The skilled artisan is also well-equipped to assess new, or additional enzymes or enzyme combinations for their utility in isolating the cells of the invention. Enzyme treatments may be 0.5, 1, 1.5, or 2 hours long or longer. In other embodiments, the tissue is incubated at approximately 37°C during the enzyme treatment of the dissociation step. Diluting the digest may also improve yields of cells as cells may be trapped within a viscous digest.

While the use of enzyme activites is disclosed, it is not required for isolation methods as provided herein. Methods based on mechanical separation alone may be successful in isolating the instant cells from the disclosed tissues.

The isolated cells can be resuspended after the tissue is dissociated into any culture medium as discussed herein above. Cells may be resuspended following a centrifugation step to separate out the cells from tissue or other debris. Resuspension may involve mechanical methods of resuspending, or simply the addition of culture medium to the cells. Growth conditions provided herein allow for a wide range of options as to culture medium, supplements, atmospheric conditions, and relative humidity for the cells. A suitable culture temperature is approximately 37°C, however the temperature may range from about 35°C to 39°C depending on the other culture conditions and desired use of the cells or culture. Also provided are methods wherein the cells can expand in the presence of from about 5% to about 20% oxygen in their atmosphere. Methods to obtain cells that require L-valine require that cells be cultured in the presence of L-valine. After a cell is obtained, its need for L-valine can be tested and confirmed by growing on D-valine containing medium that lacks the L-isomer.

Methods are provided wherein the cells can undergo at least 25, 30, 35, or 40 doublings prior to reaching a senescent state. Methods for deriving cells capable of doubling to reach 1x10¹⁴ cells or more are provided. Preferred are those methods which derive cells that can double sufficiently to produce at least about 1x10¹⁴, 1x10¹⁵, 1x10¹⁶, or 1x10¹⁷ or more cells when seeded at from about 1x10³ to about 1x10⁶ cells/cm² in culture. Preferably these cell numbers are produced within 80, 70, or 60 days or less.

In particular embodiments, tissue fibroblast cells are isolated and expanded, and possess one or more markers selected from a group comprising of CD10, CD13, CD44, CD73, CD90, CD141, PDGFr-alpha, CD105, CDXC-4, HLA-A, HLA-B, or HLA-C. In addition, the cells do not produce one or more of CD31, CD45, CD117, CD141, HLA-DR, HLA-DP, or HLA-DQ.

In some embodiments of the disclosure, genetic modification of fibroblasts is performed to cause reduction of immunogenicity of the fibroblasts. One method provides for genetic modification that includes cytoplasmic transfer with cells possessing reduced immunogenicity, such as immature dendritic cells. In another embodiment, gene editing is utilized to selectively excise inflammation-evoking genes, such as HLA or costimulatory molecules such as CD40, CD80, CD86, TNF-alpha, HMGB-1, IFN-gamma, IL-1 beta, IL-17, FAP, IL-18, IL-33, or a combination thereof.

In particular embodiments of the disclosure, one or more immunomodulatory agent(s) are expressed in universal donor fibroblasts *via* a recombinant expression vector operable in eukaryotic cells, and the expression of the immunomodulatory agent(s) may be regulated by a constitutive promoter or an inducible promoter or a tissue-specific promoter. In specific embodiments, the vector is a viral vector, such as a retrovirus, lentivirus, adenovirus, adeno-associated virus, or herpes simplex virus, or the vector is a non-viral vector, such as naked DNA or plasmid DNA or minicircle DNA. Polynucleotides of particular interest for transfection into the fibroblasts include at least the following: Fas ligand, TGF-beta, IL-4, IL-10, HLA-G, indolamine 2,3 deoxygenase (IDO), galectin family members, Galectin 3, arginase, IL-20, HGF, PDGF-BB, EGF, IGF, GDF-5, GDF-11, Angiopoietin, FGF-1, FGF-2, FGF-5, FGF-15, or a combination thereof. In specific cases, recombinantly expressed angiogenic agent(s) may comprise FAS ligand, IL-2, IL-4, IL-10, IL-20, IL-35, HLA-G, I-309, IDO, iNOS, CD200, Galectin 3, arginase, PGE-2, TGF-beta, CTLA-4, PD-L1, IFN-gamma, or combinations thereof.

In certain embodiments a therapeutically effective amount of modified cells are co-administered with one or more immunomodulatory agents(s) to an individual. Exemplary immunomodulatory agents may comprise FAS ligand, IL-2R, IL-1 Ra, IL-2, IL-4, IL-8, IL-10, IL-20, IL-35, HLA-G, PD-L1, I-309, IDO, iNOS, CD200, Galectin 3, sCR1, arginase, PGE-2, aspirin, atorvastatin, fluvastatin, lovastatin, pravastatin, rosuvastatin, simvastatin, pitavastatin, n-acetylcysteine, rapamycin, IVIG, naltrexone, TGF-beta, VEGF, PDGF, CTLA-4, anti-CD45RB antibody, hydroxychloroquine, leflunomide, auranofin, dicyanogold, sulfasalazine, methotrexate, glucocorticoids, etanercept, adalimumab, abatacept, anakinra, certolizumab, Etanercept-szzs, golimumab, infliximab, rituximab, tocilizumab, cyclosporine, IFN-gamma, everolimus, rapamycin, or combinations thereof.

In particular embodiments, methods are employed in that cells require no exogenous growth factors, except, in at least some cases, growth factors are available in the supplemental serum provided with the growth medium. Also provided herein are methods of deriving cells capable of expansion in the absence of particular growth factors. The methods are similar to the method above, however they may require that the particular growth factors (for which the cells have no requirement) be absent in the culture medium in which the cells are ultimately resuspended and grown in. In this sense, the method is selective for those cells capable of division in the absence of the particular growth factors. Particular cells, in some embodiments, are capable of growth and expansion in chemically-defined growth media with no serum added. In such cases, the cells may require certain growth factors, which can be added to the medium to support and sustain the cells. Factors that may be added for growth on serum-free media may comprise one or more of FGF, EGF, IGF, and PDGF. In some embodiments, two, three or all four of the factors are add to serum free or chemically defined media. In specific embodiments, leukemia inhibitory factor, LIF is added to serum-free medium to support or improve growth of the cells.

According to particular embodiments, the disclosure teaches production of pharmaceutical compositions comprising fibroblasts for treating brain injury, wherein the fibroblasts have been enriched for expression of CD105. The fibroblast cells may also be enriched for CD73 and/or CXCR-4 expression. According to some embodiments, the CD105 expressing cells further comprise a subpopulation of potent SDF-1 mobile CD105/CXCR-4+ cells that have CXCR-4-mediated chemotactic activity.

In particular embodiments, chemotactic fibroblasts are prepared by isolating or purifying CD105+ fibroblast cells from a population of fibroblasts cells isolated from bone marrow, cord blood, cord tissue, placenta, or skin harvested from the subject. According to some embodiments, chemotactic fibroblasts are prepared by isolating or purifying CD105 expressing cells from a population of mononuclear cells isolated from dermal biopsy of an individual.

According to particular embodiments, chemotactic fibroblasts are prepared by isolating or purifying CD105 expressing fibroblast cells from a population of cells isolated from peripheral blood collected from the subject, after treatment with a hematopoietic stem cell mobilizing agent. According to some such embodiments, the hematopoietic stem cell mobilizing agent comprises G-CSF, GM-CSF, or a pharmaceutically acceptable analog or derivative thereof. According to some embodiments, the hematopoietic stem cell mobilizing agent is a recombinant analog or derivative of a colony stimulating factor. According to some embodiments, the hematopoietic stem cell mobilizing agent is filgrastim and/or mobilzil. According to some embodiments, the chemotactic activity of the CD105+/CXCR-4+ cells is mediated by SDF-1, VEGF, and/or CXCR-4. Furthermore, according to another embodiment, at least about 1% to at least about 95% of the CD105+ cells are viable for at least about 24, at least about 48 hours, or at least about 72 hours following acquisition.

### III. Administration of Fibroblasts

Particular embodiments of the disclosure concern the administration of fibroblasts to an individual. The cells may be prepared for administration in a pharmaceutically acceptable carrier, for example a sterile saline isotonic solution. In specific embodiments the individual has a brain injury, which may include one or more of a traumatic brain injury, a stroke, a transient ischemic attack, and/or a tauopathy. The brain injury may induce neurodegenerative effects that are ameliorated by administration of fibroblasts. In particular embodiments fibroblasts are administered systemically, but may also be administered locally to the site of injury, using any suitable delivery method in order to promote regeneration and inhibit dysfunction of axons subsequent to diffuse injury.

In particular embodiments, the fibroblasts are administered to the individual by injection, including intrathecal injection and intravenous injection. Fibroblasts may also be administered intranasally. The cells may be administered in an aerosolized form. The fibroblasts may also be administered locally to the site of injury. For example, access for administration directly to the brain may occur during surgery on an individual that has suffered a TBI. Administration in such an example may be done by delivering the cells directly to the brain tissue.

The dose of fibroblasts appropriate to be used in accordance with various embodiments of the disclosure may depend on numerous factors. It may vary considerably for different circumstances. The parameters that will determine optimal doses of placental fibroblasts to be administered for primary and adjunctive therapy generally will include some or all of the following: the disease being treated and its stage; the species of the subject, their health, gender, age, weight, and metabolic rate; the subject's immunocompetence; other therapies being administered; and expected potential complications from the subject's history or genotype. The parameters may also include: whether the fibroblasts are syngeneic, autologous, allogeneic, or xenogeneic; their potency (specific activity); the site and/or distribution that must be targeted for the fibroblasts to be effective; and such characteristics of the site such as accessibility to fibroblasts and/or engraftment of fibroblasts. Additional parameters include co-administration with fibroblasts of other factors (such as growth factors and cytokines). The optimal dose in a given situation also will take into consideration the way in which the cells are formulated, the way they are administered, and the degree to which the cells will be localized at the target sites following administration. Finally, the determination of optimal dosing necessarily will provide an effective dose that is neither below the threshold of maximal beneficial effect nor above the threshold where the deleterious effects associated with the dose of fibroblasts outweighs the advantages of the increased dose.

The optimal dose of fibroblasts for some embodiments will be in the range of doses used for autologous, mononuclear bone marrow transplantation. For fairly pure preparations of placental fibroblasts, optimal doses in various embodiments will range from 1x10⁴ to 1x10⁸ fibroblasts cells/kg of recipient mass per administration. In particular cases, the range is from 1x10⁴ to 1x10⁸, 1x10⁴ to 1x10⁷, 1x10⁴ to 1x10⁶, 1x10⁴ to 1x10^{5,} 1x10⁵ to 1x10⁸, 1x10⁵ to 1x10⁸, 1x10⁶ to 1x10⁸, or 1x10⁷ to 1x10⁸, cells/kg, for example. In some embodiments a useful dose per administration will be between 1x10⁵ to 1x10⁷ fibroblasts cells/kg. In many embodiments a useful dose per administration will be 5×10⁵ to 5×10⁶ fibroblasts cells/kg. By way of reference, higher doses in the foregoing are analogous to the doses of nucleated cells used in autologous mononuclear bone marrow transplantation. Some of the lower doses are analogous to the number of CD34⁺ cells/kg used in autologous mononuclear bone marrow transplantation.

It is to be appreciated that a single dose may be delivered all at once, fractionally, or continuously over a period of time. The entire dose also may be delivered to a single location or spread fractionally over several locations. In various embodiments, fibroblasts may be administered in an initial dose, and thereafter maintained by 1, 2, 3, 4, 5, 6, 7, 8, 9, 10, 11, 12, 13, 14, 15, 16, 17, 18, 19, 20, or more further administration of fibroblasts. Fibroblasts may be administered by one method initially, and thereafter administered by the same method or one or more different methods. The subject's fibroblasts levels can be maintained by the ongoing administration of the cells. Various embodiments administer the fibroblasts either initially or to maintain their level in the subject or both by intravenous injection, for example. In a variety of embodiments, other forms of administration, are used, dependent upon the patient's condition and other factors, discussed elsewhere herein.

It is noted that human subjects are treated generally longer than experimental animals; but, treatment generally has a length proportional to the length of the disease process and the effectiveness of the treatment. Those skilled in the art will take this into account in using the results of other procedures carried out in humans and/or in animals, such as rats, mice, non-human primates, and the like, to determine appropriate doses for humans. Such determinations, based on these considerations and taking into account guidance provided by the present disclosure and the prior art will enable the skilled artisan to do so without undue experimentation. Suitable regimens for initial administration and further doses or for sequential administrations may all be the same or may be variable. Appropriate regimens can be ascertained by the skilled artisan, from this disclosure, the documents cited herein, and the knowledge in the art.

The dose, frequency, and duration of treatment will depend on many factors, including the nature of the disease, the subject, and other therapies that may be administered. Accordingly, a wide variety of regimens may be used to administer fibroblasts. In some embodiments fibroblasts are administered to a subject in one dose. In others fibroblasts are administered to a subject in a series of two or more doses in succession. In some other embodiments, wherein fibroblasts are administered in a single dose, in two doses, and/or more than two doses, the doses may be the same or different, and they are administered with equal or with unequal intervals between them.

Fibroblasts may be administered in many frequencies over a wide range of times. In some embodiments, fibroblasts are administered over a period of less than one day. In other embodiment they are administered over two, three, four, five, or six days. In some embodiments fibroblasts are administered one or more times per week, over a period of 1, 2, 3, 4, 5, 6, 7, 8, 9, 10, 11, 12, or more weeks. In other embodiments they are administered over a period of weeks for one to 2, 3, 4, 5, 6, 7, 8, 9, 10, 11, or 12 months. In various embodiments they may be administered over a period of 1, 2, 3, 4, 5, 6, 7, 8, 9, 10, 11, or 12 months. In others they may be administered over a period of 1, 2, 3, 4, 5, 6, 7, 8, 9, 10, or more years. Generally lengths of treatment will be proportional to the length of the disease process, the effectiveness of the therapies being applied, and the condition and response of the subject being treated.

In particular embodiments of the disclosure, circulating levels of one or more biomarkers are used to determine amount of fibroblasts to be administered for treatment of brain injury. One such biomarker that may be used is the Tau protein. Tau is a structural protein with six isoforms in humans and is a normal constituent of axons. Four distinct isoforms of tau may be applied in biomarker measurements; total-tau (t-tau), cleaved microtubule-associated tau (c-tau), phosphorylated tau (p-tau), and tau-A. Tau has been linked to axonal damage following TBI. Specifically, the presence of c-tau in CSF is a highly sensitive indicator of axonal injury [14, 15]. Chronic or acute altered levels of Tau have also been shown to indicate the presence of a tauopathy. The levels or amount of tau in an individual may be measured using any known method in the art, including ELISA, mass spectrometry, HPLC, LC/MS, Western blotting, immunoprecipitation, immunostaining, immunoelectrophoresis, for example. Tau may be measured in any biological sample from the individual, including blood, plasma, CSF, ISF, or a combination thereof.

In particular embodiments, fibroblasts may be administered in combination with another therapy to treat a medical condition, such as a stroke or acute ischemic attack. Fibroblasts may be administered during supportive therapies such physical therapies, cognitive therapies, communication therapies, brain stimulating therapies, or a combination thereof.

In particular embodiments, fibroblasts may be administered in combination with one or more other therapies to treat or prevent a brain injury. Fibroblasts may be administered during at least one medical procedure, for example, a surgery, such as surgery aimed to remove clotted blood, repair skull fractures, reduce bleeding in the brain, relieve pressure in the skull, or a combination thereof. Fibroblasts may be administered with at least one therapy to induce a coma, which may be given to reduce stress and/or swelling in the brain during recovery. Fibroblasts may be administered with one or more other medications useful for an individual recovering from a brain injury including anti-anxiety agents, anticoagulants, anticonvulsants, antidepressants, diuretics, muscle relaxants, stimulants, or a combination thereof. Fibroblasts may be administered in combination with at least one supportive therapy, such as physical therapies, occupational therapies, recreational therapies, speech or communication therapies, psychological counseling, vocational counseling, cognitive therapies, or a combination thereof.

In particular embodiments, fibroblasts may be administered in combination with another therapy to treat a medical condition, such as a tauopathy. Fibroblasts may be administered in combination with an agent that inhibits tau, the accumulation of tau, the misfolding of tau, and/or the formation of Tau inclusions. The agents may be a vaccine for Tau pathologies (examples include AADvac-1, ACI-35), an antibody against Tau or Tau related proteins (examples include BMS-986168, ABBV-8E12), a microtubule stabilizer (examples include TPI-287), a Tau aggregate inhibitor (examples include TRx0237), an agent that inhibits post-translational modifications of Tau (examples include agents that inhibit Tau phosphorylation (such as lithium chloride, tideglusib), agents that inhibit Tau o-linked glycosylation (such as, thiamet-G), agents that inhibit Tau acetylation (such as, salsalate)), Hsp90 inhibitors or a combination thereof. Provided examples of agents that modulate Tau or Tau related mechanisms may have non-Tau related activities and therefore their classification and mechanisms of action are only provided as examples, which are not limiting in their intended use, either alone or in combination with the administration of fibroblasts disclosed herein.

### IV. Brain Injuries

Particular embodiments of the present disclosure concern the methods of administering fibroblasts to an individual with a brain injury, such as wherein the brain injury is a traumatic brain injury (TBI). Examples of a TBI include chronic traumatic encephalopathy, one or more concussions, blast-induced traumatic brain injury, coup-contrecoup brain injury, brain contusion, shaken baby syndrome, a penetrating injury, diffuse axonal injury, second impact injury, locked-in syndrome, or a combination thereof. The TBI may range in severity and may present with varying symptoms. In some embodiments, the individual may or may not have more than one TBI, which may or may not range in severity. At least one of the injuries may or may not have gone undiagnosed. The event that causes the TBI may be from a direct or indirect impact to the body of the individual. The individual may or may not have received one or more TBIs from at least one impact to the head, including for example one or more blunt impacts and/or one or more piercing impacts. The individual also may have received one or more TBIs from whiplash, such as from a car accident or resulting from shaken baby syndrome.

Particular embodiments concern the administration of fibroblasts to an individual with a brain injury, wherein the brain injury is a stroke or a transient ischemic attack (TIA). The stroke may be an ischemic stroke or may be a hemorrhagic stroke. The administration of the fibroblasts may occur at the time of diagnosis for the stroke or TIA or at a time subsequent the diagnosis. The subsequent time of administration of the fibroblasts may be minutes, hours, days, weeks, or years after the stroke (or TIA) or an initial diagnosis. The fibroblasts may be administered concurrently with at least one other stroke or TIA therapy, in order for the fibroblasts to act additively or synergistically with the other therapy or therapies. In some embodiments, the individual is administered fibroblasts prior to diagnosis, wherein the individual may or may not be determined to be at risk for having a stroke or TIA. An individual suspected of having a stroke may be provided an effective amount of the fibroblasts

In some embodiments of the disclosure, fibroblasts are administered to an individual, wherein the individual has a tauopathy. The tauopathy may be an acute tauopathy or may be a chronic tauopathy. The acute tauopathy may be TBI, which may include diffuse axonal injury, concussion, contusion, Coup-Contrecoup injury, Second Impact Syndrome, penetrating injury, Shaken Baby Syndrome, and Locked In Syndrome. In any of the embodiments described above or herein, the acute tauopathy can be stroke. In any of the embodiments described above or herein, the acute tauopathy can be chronic traumatic encephalopathy. The tauopathy may also be a chronic tauopathy, which is further defined herein. The tauopathy may or may not be diagnosed, as the individual may be suspected of having a taopthy and may or may not have a personal or family history.

### EXAMPLES

The following examples are included to demonstrate preferred embodiments of the disclosure. It should be appreciated by those of skill in the art that the techniques disclosed in the examples which follow represent techniques discovered by the inventor to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the disclosure.

### EXAMPLE 1

### FIBROBLASTS ARE SUPERIOR TO MSC AT DECREASING STROKE-INDUCED NEUROLOGICAL DEFICITS

For the studies, female Sprague-Dawley rats (Charles River Laboratories, Inc., Wilmington, MA) weighing 260-310 g were used. The animals in the experimental groups were allocated in a randomized fashion. Investigators were blinded to dose and treatment group allocation during surgery and during outcome evaluations. The animals underwent Reversible Middle cerebral artery occlusion (rMCAo) following an overnight fast. Anesthesia was induced with 3% isofluorane and 70% nitrous oxide. Rats were intubated endotracheally and ventilated mechanically on a mixture of 1-0.5% isofluorane, 70% nitrous oxide and a balance of oxygen. The right femoral artery and vein were catheterized to permit to monitor blood pressure and to take arterial samples for blood gas and glucose assessments. Arterial *P*CO₂ and *P*O₂ were maintained in the normal range by ventilator adjustment. For immobilization, rats received pancuromium bromide 0.75 mg/kg i.v. Rectal temperature was measured continuously and maintained at 37-37.5°C by a heating pat under the rat's body. Cranial temperature was separately monitored by a 29-gauge thermocouple implanted into the right temporalis muscle and was maintained at 36-36.5°C by a warming lamp placed above the rat's head throughout the experiment.

The common carotid artery (CCA) was exposed through a midline incision on the ventral neck and carefully separated from the surrounding tissue including the adjacent vagus nerve by blunt dissection using microsurgery. Under an operation microscope, the branches of external carotid artery (ECA), superior thyroid and ascending pharyngeal arteries and further the terminal lingual and maxillary artery branches, were dissected and coagulated. Two long 5-0 silk sutures tied the ECA as distal to carotid bifurcation as possible. Two short (3 cm) 5-0 silk sutures were tied loosely around the segment of ECA closed to the bifurcation. The ICA was exposed, and the origin of the pterygopalatine artery (PPA) was visualized. An intraluminal 3.0 nylon suture, having a spherical and enlarged tip produced by heating near flame, introduced into the distal segment of ECA *via* a small incision on the vessel and advanced into the ECA lumen to reach the bifurcation. The 2 short silk sutures subsequently were tied to prevent bleeding. The ECA was cut at the distal segment between the 2 long-suture notes. The stump of the ECA was held toward the surgeon; thus the stump and the ICA were on a straight line. The suture was then turned into ICA with a special care not enter the PPA. The suture went advance in ICA until the sharp drop of regional cerebral blood flow confirmed by the LDF. The length of inserted suture inside the ICA from the bifurcation was 19-25 mm. A sudden and sharp decrease in the LDF signal was interpreted to indicate a successful MCA occlusion. The suture was gently withdrawn after the 90-min occlusion period. During the procedure and the 2-h recirculation, blood pressure and the blood gas were maintained in the normal ranges, cranial and rectal temperature were maintained at normal levels

A standardized neurobehavioral test battery was conducted as described previously (Ley JJ, Vigdorchik A, Belayev L, Zhao W, Busto R, et al. (2005) Stilbazulenyl nitrone, a second-generation azulenyl nitrone antioxidant, confers enduring neuroprotection in experimental focal cerebral ischemia in the rat: neurobehavior, histopathology, and pharmacokinetics. J Pharmacol Exp Ther 313: 1090-1100)., which includes tests for postural reflex, sensorimotor integration and proprioception. Total neurological score ranged from a normal score of 0 to a maximal possible score of 12.

Foreskin fibroblasts where obtained from AllCells and cultured according to manufacturer's instructions with the exception that purification of CD73-expressing fibroblasts was performed prior to administration into animals. Selection of CD73-expressing cells was performed using magnetic activated sorting (MACS) (Milteny) according to the manufacturer's instructions. Mesenchymal stem cells were obtained from AllCells and cultured according to manufacturer's instructions. Both MSC and fibroblasts were administered at a concentration of 1 million cells per animal, intravenously, 24 hours subsequent to middle cerebral artery ligation.

As seen in FIG. 1, fibroblast administration was associated with superior neurological recovery, as compared to MSC.

### REFERENCES

The following references, to the extent that they provide exemplary procedural or other details supplementary to those set forth herein, are specifically incorporated herein by reference.
1. Feigin, V.L., et al., Global and regional burden of stroke during 1990-2010: findings from the Global Burden of Disease Study 2010. Lancet, 2014. 383(9913): p. 245-54.
2. Johnston, S.C., S. Mendis, and C.D. Mathers, Global variation in stroke burden and mortality: estimates from monitoring, surveillance, and modelling. Lancet Neurol, 2009. 8(4): p. 345-54.
3. Chamorro, A., et al., Neuroprotection in acute stroke: targeting excitotoxicity, oxidative and nitrosative stress, and inflammation. Lancet Neurol, 2016. 15(8): p. 869-881.
4. Del Zoppo, G.J., et al., Expansion of the time window for treatment of acute ischemic stroke with intravenous tissue plasminogen activator: a science advisory from the American Heart Association/American Stroke Association. Stroke, 2009. 40(8): p. 2945-8.
5. Saver, J.L., et al., Time to Treatment With Endovascular Thrombectomy and Outcomes From Ischemic Stroke: A Meta-analysis. JAMA, 2016. 316(12): p. 1279-88.
6. Jin, R., et al., Role of inflammation and its mediators in acute ischemic stroke. J Cardiovasc Transl Res, 2013. 6(5): p. 834-51.
7. Tsitsopoulos, P.P., S. Abu Hamdeh, and N. Marklund, Current Opportunities for Clinical Monitoring of Axonal Pathology in Traumatic Brain Injury. Front Neurol, 2017. 8: p. 599.
8. Leetch, A.N. and B. Wilson, Pediatric Major Head Injury: Not a Minor Problem. Emerg Med Clin North Am, 2018. 36(2): p. 459-472.
9. Carothers, C., et al., Activated prothrombin complex concentrate for warfarin reversal in traumatic intracranial hemorrhage. J Surg Res, 2018. 223: p. 183-187.
10. Omalu, B., et al., Postmortem Autopsy-Confirmation of Antemortem [F-18]FDDNP-PET Scans in a Football Player With Chronic Traumatic Encephalopathy. Neurosurgery, 2018. 82(2): p. 237-246.
11. Aldag, M., et al., The Biological Basis of Chronic Traumatic Encephalopathy following Blast Injury: A Literature Review. J Neurotrauma, 2017. 34(S 1): p. S26-S43.
12. Alosco, M.L., et al., Repetitive head impact exposure and later-life plasma total tau in former National Football League players. Alzheimers Dement (Amst), 2017. 7: p. 33-40.
13. Oliver, J.M., et al., Fluctuations in blood biomarkers of head trauma in NCAA football athletes over the course of a season. J Neurosurg, 2018: p. 1-8.
14. Li, J., et al., Biomarkers associated with diffuse traumatic axonal injury: exploring pathogenesis, early diagnosis, and prognosis. J Trauma, 2010. 69(6): p. 1610-8.
15. Ost, M., et al., Initial CSF total tau correlates with 1-year outcome in patients with traumatic brain injury. Neurology, 2006. 67(9): p. 1600-4.

## Claims

1. A population of CD73 and CD105-positive fibroblasts for use in a method of inducing regeneration and/or a healing response in the brain of an individual, said method comprising administering a therapeutically effective amount of the fibroblasts to the individual, wherein the individual has a traumatic brain injury, has suffered from a stroke and/or a transient ischemic attack or has a chronic or acute tauopathy.

2. The population of CD73 and CD105-positive fibroblasts for use according to claim 1, wherein:
a) the traumatic brain injury is selected from the group consisting of chronic traumatic encephalopathy, one or more concussions, blast-induced traumatic brain injury, coup-contrecoup brain injury, brain contusion, shaken baby syndrome, a penetrating injury, diffuse axonal injury, second impact injury, locked-in syndrome, and a combination thereof; or
b) the stroke is an ischemic stroke or a hemorrhagic stroke; or
c) circulating levels of total tau, cleaved microtubule-associated tau, phosphorylated tau, and/or tau-A are measured in the individual.

3. The population of CD73 and CD105-positive fibroblasts for use according to claim 1 or 2, wherein:
a) the fibroblasts possess regenerative activity wherein the regenerative activity comprises: the ability to produce a factor selected from the group consisting of VEGF, BDNF, NGF, PDGF-BB, and a combination thereof; and/or the ability to stimulate neural stem cell proliferation; and/or
b) the fibroblasts are derived from tissues selected from the group consisting of skin, foreskin, adipose, peripheral blood, cerebral spinal fluid, bone marrow, placenta, cord blood, Wharton's jelly, and a combination thereof.

4. The population of CD73 and CD105-positive fibroblasts for use according to any one of claims 1-3, wherein the fibroblasts further express:
a) CXCR-4; or
b) CD90 and/or CXCR-4.

5. The CD73 and CD105-positive population of fibroblasts for use according to any one of claims 1-4, wherein:
a) the fibroblasts express at least one of the markers selected from the group consisting of OCT-4, NANOG, SSEA-4, stem cell factor receptor, and a combination thereof; and/or
b) the population of fibroblasts comprises fibroblasts isolated by a method comprising the steps of isolating a mammalian cell population and enriching for a subpopulation, wherein the subpopulation expresses a CD45⁻ phenotypic profile to thereby isolate fibroblasts with regenerative properties; and/or
c) the fibroblasts are administered intranasally, intrathecally, and/or intravenously; and/or
d) the fibroblasts are administered locally to the brain or systemically.

6. The CD73 and CD 105-positive population of fibroblasts for use according to any one of claims 1-5, wherein the individual is provided an additional therapy for a brain injury or stroke and/or a transient ischemic attack or chronic or acute tauopathy.

7. The CD73 and CD105-positive population of fibroblasts for use according to claim 6, wherein the additional therapy comprises at least one medical procedure, at least one agent, at least one supportive therapy, or a combination thereof.

8. The CD73 and CD105-positive population of fibroblasts for use according to claim 7, wherein:
a) the medical procedure comprises a surgery to remove clotted blood, repair skull fractures, reduce bleeding in the brain, relieve pressure in the skull, or a combination thereof; or
b) the agent is at least one agent selected from the group consisting of anti-anxiety agents, anticoagulants, anticonvulsants, antidepressants, diuretics, muscle relaxants, stimulants, agents to medically-induce a coma, and a combination thereof; or
c) the agent is at least one agent selected from the group consisting of a vaccine for Tau pathologies, an antibody against Tau or Tau related proteins, a microtubule stabilizer, a Tau aggregate inhibitor, an agent that inhibits post-translational modifications of Tau, Hsp90 inhibitors, and a combination thereof; or
d) the supportive therapy comprises at least one physical therapy, occupational therapy, recreational therapy, speech or communication therapy, psychological counseling, vocational counseling, cognitive therapy, brain stimulation therapy, or a combination thereof.

## Patentansprüche

1. Population von CD73- und CD105-positiven Fibroblasten zur Verwendung in einem Verfahren zum Induzieren von Regeneration und/oder einer Heilungsreaktion im Gehirn eines Individuums, das Verfahren umfassend ein Verabreichen einer therapeutisch wirksamen Menge der Fibroblasten an das Individuum, wobei das Individuum eine traumatische Hirnverletzung erlitten hat, einen Schlaganfall und/oder eine transitorische ischämische Attacke erlitten hat oder eine chronische oder akute Tauopathie hat.

2. Population von CD73- und CD105-positiven Fibroblasten zur Verwendung nach Anspruch 1, wobei:
a) die traumatische Hirnverletzung ausgewählt ist aus der Gruppe bestehend aus chronischer traumatischer Enzephalopathie, einer oder mehreren Gehirnerschütterungen, stoßinduzierter traumatischer Hirnverletzung, Hirnkontusion, Schütteltrauma, einer penetrierenden Verletzung, diffuser axonaler Verletzung, Verletzung durch zweiten Aufprall, Locked-in-Syndrom und einer Kombination davon; oder
b) der Schlaganfall ein ischämischer Schlaganfall oder ein hämorrhagischer Schlaganfall ist; oder
c) zirkulierende Werte von Gesamt-Tau, gespaltenem Mikrotubuli-assoziiertem Tau, phosphoryliertem Tau und/oder Tau-A bei dem Individuum gemessen werden.

3. Population von CD73- und CD105-positiven Fibroblasten zur Verwendung nach Anspruch 1 oder 2, wobei:
a) die Fibroblasten eine regenerative Aktivität besitzen, wobei die regenerative Aktivität Folgendes umfasst: die Fähigkeit, einen Faktor zu produzieren, der ausgewählt ist aus der Gruppe, bestehend aus VEGF, BDNF, NGF, PDGF-BB und einer Kombination davon; und/oder die Fähigkeit, Proliferation neuraler Stammzellen zu stimulieren; und/oder
b) die Fibroblasten von Gewebe abgeleitet sind, die ausgewählt sind aus der Gruppe, bestehend aus Haut, Vorhaut, Fettgewebe, peripherem Blut, zerebraler Rückenmarksflüssigkeit, Knochenmark, Plazenta, Nabelschnurblut, Wharton-Gelee und einer Kombination davon.

4. Population von CD73- und CD105-positiven Fibroblasten zur Verwendung nach einem der Ansprüche 1-3, wobei die Fibroblasten ferner Folgendes exprimieren:
a) CXCR-4; oder
b) CD90 und/oder CXCR-4.

5. CD73- und CD105-positive Population von Fibroblasten zur Verwendung nach einem der Ansprüche 1-4, wobei:
a) die Fibroblasten mindestens einen der Marker exprimieren, die ausgewählt sind aus der Gruppe, bestehend aus OCT-4, NANOG, SSEA-4, Stammzellfaktor-Rezeptor und einer Kombination davon; und/oder
b) die Population von Fibroblasten Fibroblasten umfasst, die durch ein Verfahren isoliert werden, umfassend die Schritte eines Isolierens einer Säugetierzellpopulation und Anreicherns auf eine Subpopulation, wobei die Subpopulation ein phänotypisches CD45⁻-Profil exprimiert, um dadurch Fibroblasten mit regenerativen Eigenschaften zu isolieren; und/oder
c) die Fibroblasten intranasal, intrathekal und/oder intravenös verabreicht werden; und/oder
d) die Fibroblasten dem Gehirn lokal oder systemisch verabreicht werden.

6. CD73- und CD105-positive Population von Fibroblasten zur Verwendung nach einem der Ansprüche 1-5, wobei der Person eine zusätzliche Therapie für eine Hirnverletzung oder einen Schlaganfall und/oder eine transitorische ischämische Attacke oder eine chronische oder akute Tauopathie verabreicht wird.

7. CD73- und CD105-positive Population von Fibroblasten zur Verwendung nach Anspruch 6, wobei die zusätzliche Therapie mindestens ein medizinisches Verfahren, mindestens ein Mittel, mindestens eine unterstützende Therapie oder eine Kombination davon umfasst.

8. CD73- und CD105-positive Population von Fibroblasten zur Verwendung nach Anspruch 7, wobei:
a) das medizinische Verfahren einen chirurgischen Eingriff zum Entfernen von Blutgerinnseln, zum Beheben von Schädelfrakturen, zum Reduzieren von Hirnblutungen, zum Entlasten von Druck im Schädel oder eine Kombination davon umfasst; oder
b) das Mittel mindestens ein Mittel ist, das ausgewählt ist aus der Gruppe, bestehend aus Anti-Angstmitteln, Antikoagulantien, Antikonvulsiva, Antidepressiva, Diuretika, Muskelrelaxantien, Stimulantien, Mitteln zum medizinischen Induzieren eines Komas und einer Kombination davon; oder
c) das Mittel mindestens ein Mittel ist, ausgewählt ist aus der Gruppe, bestehend aus einem Impfstoff für Tau-Pathologien, einem Antikörper gegen Tau oder Tau-verwandte Proteine, einem Mikrotubuli-Stabilisator, einem Tau-Aggregat-Hemmer, einem Mittel, das posttranslationale Modifikationen von Tau hemmt, Hsp90-Hemmern und einer Kombination davon; oder
d) die unterstützende Therapie mindestens eine physikalische Therapie, Beschäftigungstherapie, Freizeittherapie, Sprach- oder Kommunikationstherapie, psychologische Beratung, Berufsberatung, kognitive Therapie, Hirnstimulationstherapie oder eine Kombination davon umfasst.

## Revendications

1. Population de fibroblastes CD73 et CD105 positifs pour une utilisation dans un procédé d'induction de réponse de régénération et/ou de cicatrisation dans le cerveau d'un individu, ledit procédé comprenant l'administration d'une quantité thérapeutiquement efficace des fibroblastes à l'individu, dans laquelle l'individu présente une lésion cérébrale traumatique, a subi un accident vasculaire cérébral et/ou un accident ischémique transitoire ou est atteint d'une tauopathie chronique ou aiguë.

2. Population de fibroblastes CD73 et CD105 positifs pour une utilisation selon la revendication 1, dans laquelle :
a) la lésion cérébrale traumatique est sélectionnée dans le groupe constitué par une encéphalopathie traumatique chronique, une ou plusieurs commotions, une lésion cérébrale traumatique induite par explosion, une lésion cérébrale par coup-contrecoup, une contusion cérébrale, le syndrome du bébé secoué, une lésion pénétrante, une lésion axonale diffuse, une lésion de second impact, le syndrome d'enfermement, et une combinaison de ceux-ci ; ou
b) l'accident vasculaire cérébral est un accident vasculaire cérébral ischémique ou un accident vasculaire cérébral hémorragique ; ou
c) les taux en circulation de tau totale, de tau associée aux microtubules clivée, de tau phosphorylée, et/ou de tau-A sont mesurés chez l'individu.

3. Population de fibroblastes CD73 et CD105 positifs pour une utilisation selon la revendication 1 ou 2, dans laquelle :
a) les fibroblastes possèdent une activité régénérative dans laquelle l'activité régénérative comprend : la capacité à produire un facteur sélectionné dans le groupe constitué par VEGF, BNDF, NGF, PDGF-BB, et une combinaison de ceux-ci ; et/ou la capacité à stimuler la prolifération de cellules souches neuronales ; et/ou
b) les fibroblastes sont dérivés de tissus sélectionnés dans le groupe constitué par la peau, le prépuce, le tissu adipeux, le sang périphérique, le liquide céphalo-rachidien, la moelle osseuse, le placenta, le cordon ombilical, la gelée de Wharton, et une combinaison de ceux-ci.

4. Population de fibroblastes CD73 et CD105 positifs pour une utilisation selon l'une quelconque des revendications 1 à 3, dans laquelle les fibroblastes expriment en outre :
a) CXCR-4 ; ou
b) CD90 et/ou CXCR-4.

5. Population de fibroblastes CD73 et CD105 positifs pour une utilisation selon l'une quelconque des revendications 1 à 4, dans laquelle :
a) les fibroblastes expriment au moins l'un des marqueurs sélectionnés dans le groupe constitué par OCT-4, NANOG, SSEA-4, un récepteur de facteur de cellules souches, et une combinaison de ceux-ci ; et/ou
b) la population de fibroblastes comprend des fibroblastes isolés par un procédé comprenant les étapes consistant à isoler une population de cellules de mammifère et effectuer un enrichissement par une sous-population, dans laquelle la sous-population exprime un profil phénotypique CD45⁻ afin d'ainsi isoler des fibroblastes ayant des propriétés régénératives ; et/ou
c) les fibroblastes sont administrés par voie intra-nasale, intrathécale, et/ou intraveineuse ; et/ou
d) les fibroblastes sont administrés localement au cerveau ou par voie systémique.

6. Population de fibroblastes CD73 et CD105 positifs pour une utilisation selon l'une quelconque des revendications 1 à 5, dans laquelle l'individu reçoit une thérapie supplémentaire pour un lésion cérébrale ou un accident vasculaire cérébral et/ou un accident ischémique transitoire ou une tauopathie chronique ou aiguë.

7. Population de fibroblastes CD73 et CD105 positifs pour une utilisation selon la revendication 6, dans laquelle la thérapie supplémentaire comprend au moins une procédure médicale, au moins un agent, au moins une thérapie de soutien, ou une combinaison de ceux-ci.

8. Population de fibroblastes CD73 et CD105 positifs pour une utilisation selon la revendication 7, dans laquelle :
a) la procédure médicale comprend une intervention chirurgicale pour éliminer un caillot sanguin, réparer des fractures du crâne, réduire le saignement dans le cerveau, atténuer la pression dans le crâne, ou une combinaison de ces actions ; ou
b) l'agent est au moins un agent sélectionné dans le groupe constitué par les agents anxiolytiques, les anticoagulants, les anticonvulsifs, les antidépresseurs, les diurétiques, les relaxants musculaires, les stimulants, les agents permettant d'induire médicalement un coma, et une combinaison de ceux-ci ; ou
c) l'agent est au moins un agent sélectionné dans le groupe constitué par un vaccin pour les pathologies Tau, un anticorps dirigé contre Tau ou les protéines associées à Tau, un stabilisant de microtubules, un inhibiteur d'agrégats Tau, un agent qui inhibe les modifications post-translationnelles de Tau, les inhibiteurs Hsp90, et une combinaison de ceux-ci ; ou
d) la thérapie de soutien comprend au moins une thérapie physique, une ergothérapie, une ludothérapie, une thérapie par la parole ou la communication, un accompagnement psychologique, un accompagnement en orientation professionnelle, une thérapie cognitive, une thérapie par stimulation cérébrale, ou une combinaison de ceux-ci.
